# EUROPEAN PATENT SPECIFICATION

(11) **EP 1 861 075 B1**
(45) Date of publication and mention of the grant of the patent: **16.05.2012**
(21) Application number: 06792448.0
(22) Date of filing: 15.03.2006
(51) Int. Cl.: A61K 9/50

(54) **COATED GRANULAR FORMULATIONS**
BESCHICHTETE GRANULATFORMULIERUNGEN
FORMULATIONS GRANULAIRES REVETUES

(30) Priority: 23.03.2005 IT MI20050485
(43) Date of publication of application: 05.12.2007
(73) Proprietor: Graal S.r.l., 20052 Monza (IT)
(72) Inventor: SENECI, Alessandro, I-20090 Segrate (Milano) (IT); GIOVANNONE, Daniele, I-03100 Frosinone (IT); ZIO, Cesare, I-20138 Milano (IT)
(74) Representative: Pistolesi, Roberto
(86) International application number: PCT/EP2006/060761
(87) International publication number: WO 2007/003447

(56) References cited:
- EP-A- 0 421 581
- EP-A1- 0 253 684
- EP-A1- 0 588 707
- WO-A-03/043608
- DE-C1- 3 721 721
- US-A1- 2002 164 369

## Description

The present invention relates to pharmaceutical, dietary and/or foodstuff formulations, preferably in the form of granules and/or microgranules, characterized by being completely coated so that they are simultaneously protected from atmospheric agents (light, humidity, oxygen, etc.), from degenerative interactions due to physical contact with other components present in the same formulation and from the action of gastric juices (controlled release in the different sections of the digestive tract (targeting)) and so that they exercise a barrier effect in the presence of any unpleasant tastes and/or odours of the active component contained therein.

The granular formulations of the invention are used by oral administration of one or more active components coated with melted hydrogenated fats, the latter being added, if desired, together with surfactants miscible therewith and applied in a repeatable manner. In addition, colorants, pigments, opacifiers, flavouring compounds and sweeteners can be added to the fats.

In particular, the preparation of the granules of the invention, coated with one or more low-melting hydrogenated vegetable fats, has arisen initially from the need to develop a form suitable for protecting a product, designed for oral administration, which is particularly sensitive to the abovementioned agents in addition to being characterized by particularly aggressive and unpleasant organoleptic properties such as S-adenosylmethionine (SAM) and/or salts thereof and/or combinations thereof currently used in pharmaceutical preparations.

In the prior art, the coating of solid oral formulations, and in particular of powders and granules, is carried out by means of hydrophobic or hydrophilic substances (waxes, oils, methacrylic acid polymers or natural polymers, cellulose, shellac, etc.), using traditional fluid-bed systems (microencapsulation, etc.), in a mixing pan (film coating of tablets, pellets, capsules, etc.) or in a fluid-bed (tangential spray) rotor granulator in which the process parameters (temperature, flow rate, inlet air volume, etc.) require a strict control of said parameters (Ryushi Sekkei and Seizai Gijutsu, Yakugyo Jihosha, pp. 130-132, 30 October 1994).

Such customary working steps make it possible to prepare gastroresistent formulations characterized by release characteristics which allow the resorption of the active component at defined sites of the organism, where said active component exhibits its major therapeutic effect.

The methods customarily known for the preparation of coated pharmaceutical and/or dietary controlled-release formulations are described below:
a) Mixing the active component and the excipients with fats of various types and compressing the final mixture; the resulting tablets allow a prolonged release of the active components contained therein in the organism; this method is described in EP1225876, which is incorporated herein by way of reference;
b) Covering the mixtures of active components in a fluid bed with low melting point waxes; this method is described in WO97/03656, which is incorporated herein by way of reference;
c) Coating the tablets with gastroresistent lacquers (ethyl cellulose, cellulose acetophthalate, lac gum, etc.) and final surface-coating thereof with sugars;
d) Tablets with retardants: these are sugary cores on which the active component is dispersed, followed by a protective functional lacquer coating;
e) Tablets in which retardants are dispersed in such a way that part of the active component is contained in the gastroresistent retardants and part in the water-dispersible tablet.

In general, these are formulations in which the actual effect of controlling the release of the active component is based on the use of excipients and/or adjuvants external to the organism, either human or animal, in an attempt to control the resorption of the active component that takes place according to a particular kinetics thereof, without taking into account the normal physiological digestive processes. The use of such substances is often not very desirable because the resorption of the active component is not obtained according to a kinetic profile that is as close as possible to the normal digestive processes in man and/or animal.

Consequently, the abovementioned customary methods are limited by the fact that the excipients used must not be present in high percentages since they have "non-physiological" characteristics or kinetic resorption profiles which differ greatly from the physiological profiles of man and/or animal.

Moreover, the customary methods are not capable of overcoming all the formulating difficulties which often simultaneously characterize a single active component, making it quite difficult to process, such as, for example, its considerable hygroscopicity, its ease of oxidation in air, its poor palatability and its considerable instability upon contact with other substances.

In addition to that, the use of the abovementioned formulation methods is often limited by the fact that many of them require long preparation times and/or rather bulky and costly machinery.

A new gastroresistent formulation in the form of granules and/or microgranules coated with a hydrogenated vegetable fat having a low melting point has now been found and forms the object of the present invention.

The granules consisting of one or more active components are initially obtained by the normal granulation methods and can, if desired, be mixed with the more customary excipients and/or adjuvants in this field pharmacologically inert in the form of diluents, binders, lubricants, etc. and intended for low-dosage administration of the active components. Said excipients and/or adjuvants may be added in amounts of between 2 and 10% by weight, preferably between 3 and 5% by weight.

The fat is then applied, melted, to the initial granules of active component in rotating screw-type powder mixers equipped with lump-breaking blades, taking all necessary measures to prevent the formation of oily agglomerates and mixes.

If desired, it is possible to add, to the fat, in order to control better the mechanics of release in the digestive tract, surfactants which are miscible therewith homogeneously in the liquid phase.

Moreover, to ensure the subsequent functionality of the final product, pH-dependent materials can be added to the granules.

Finally, it is possible to use antiadhesives such as, for example, silica, talc, magnesium stearate, etc.

The hydrogenated vegetable fats used in the invention are characterized by being low-melting, namely having a melting point in the range of between 30° and 100°C, preferably between 50° and 80°C.

Examples of vegetable fats suitable for the present invention include: mono-, di- or triglyceryl behenates, glyceryl palmitostearate, polyethylene glycol esters of fatty acids, waxes such as, for example, carnauba wax and bees wax, cocoa butter, etc.

In particular, hydrogenated fatty acids or mixtures thereof having chains of 14 to 22 carbon atoms are used in the formulations of the invention.

The surfactants which may be added, if desired, are characterized by being miscible homogeneously with the melted fat phase. Examples of said surfactants include: polysorbates in general, poloxamers, di- and triethanolamine, glyceryl monostearate, etc. In particular, in the present invention it is possible to use soya lecithin and polysorbates 60, 65 and 80 in concentrations ranging from 0.1% to 10% by weight, more frequently from 0.5% to 5.0%.

The pH-dependent substances which may be added, if desired, in the final stage of mixing are powders that are dispersible in melted fats such as: Eudragit L100-55, cellulose acetophthalate, amides that are advantageously modified. In particular, in the present invention it is possible to use different shellac salts or alcoholic shellac and hydroxypropylmethylcellulose (methocel).

The hydrogenated vegetable fats are used in a percentage of between 10% and 50 % by weight, preferably between 20% and 40 % by weight, relative to the weight of the formulation.

The active component contained in the granular formulations of the present invention is S-adenosylmethionine (SAMe) or its salts established in pharmaceutical practice and/or their mixtures.

The pharmaceutically used salts of SAMe of the present invention may be chosen from among: disulphate, ditosylate, para-toluenesulphonate, 1,4-butanedisulphonate and/or hexamethanephosphonate.

The active component or components are contained in the granular formulations of the present invention in a percentage ranging from 50% to 90% by weight of the formulation, preferably from 60% to 80%.

Therefore, one of the main objects of the present invention is represented by a granular formulation based on granules formed by an inner core essentially consisting of at least one active component or a pharmaceutically acceptable salt thereof in an amount of between 50% and 90% by weight of the formulation, and by an outer coating essentially consisting of hydrogenated vegetable fats in an amount of between 10% and 50 % by weight of the formulation.

The preferably used active components are non-palatable, hygroscopic, oxidizable and/or photosensitive active components. In particular, for the purposes of the present invention, the term "hygroscopic" is understood as meaning an active component which is able to absorb rapidly an amount of water of between 3% and 40% by weight and furthermore, in the case of "deliquescent" products, is usually characterized, after a short exposure to ambient air at moderate climates, by humidity levels which range from 50% to 75% and by temperatures in the range of 20° to 35°C.

Furthermore, for the purposes of the present invention, the term "essentially consisting of" is understood as meaning that the granular formulation can contain, in addition to the active component and the hydrogenated vegetable fats, minimum quantities (not exceeding 10% of the weight of the formulation) of excipients and/or pharmaceutically compatible adjuvants.

A particular application of the granular formulation of the present invention is that it allows the administration to livestock of active components for veterinary use, such as, for example, SAMe, mixing it with the feed, thus preventing the active component from becoming modified by contact with the feed or by exposure to atmospheric agents, or from being rejected by the animal due to its poor palatability. Therefore, a second object of the present invention is the use of the abovementioned granular formulation for preparing mixtures prescribed for veterinary use.

Furthermore, another object of the present invention is a process for the production of the coated gastroresistent granules described above. In said process, the starting materials are used in the form of powders with a wide range of particle sizes; in the first process stages, the powders are subjected to a granule-forming process in which the granules are characterized by having an average particle size of between 250 and 3000 µ, in particular, between 250 and 1000 µ.

In particular, the process according to the invention comprises the following stages:
1^{st} stage: formation of a granulate by mixing according to known methods; in particular, the granulate is formed, for example, by compacting the powder mixtures by means of compression (compressing machines, compactors or the like).
2^{nd} stage: breaking up the slugs into granules of sizes between 250 and 3000 p, more frequently between 500 and 1000 p. The desired dimensions can be obtained by using meshes or plates with calibrated holes (vibrating granulators of the Viani, Manes or other type).
3^{rd} stage: sieving the granulate produced, in order to eliminate the fines (below 250 µ), selecting the desired fraction.
4^{th} stage: charging the selected granulate into suitable mixers (Diosna type, etc.), preferably into rotating screw mixers and, more preferably, mixers of the SAGA type with dimensions such that they are able to contain between 100 and 1000 kg of product, preferably between 200 and 500 kg. The mixer must be temperature-regulated, so as to ensure operation at a constant temperature during the entire process.
5^{th} stage: coating the granulate with melted hydrogenated fat, if desired, with the addition of surfactants miscible in the oily liquid. The addition of the melt is carried out continuously by means of a peristaltic pump, preferably by means of an airless atomizer at a flow rate of between 50 e 2000 g/min, preferably between 200 and 1000 g/min.
   The temperature of the mass to be coated is between 20° and 60°C, preferably between 35° and 55°C.
6^{th} stage: if desired, coating the granulate obtained in stage 5 with pH-dependent pulverulent substances, using again the previously selected mixer. These substances are added in amounts of between 2% and 10 % by weight, preferably between 3% and 5 %.
7^{th} stage: separating off, for example on a shaking sieve, any agglomerates which may have formed during the coating.

In the preparation of the granulate according to stages 1 and 2, it is possible to envisage the use of customary excipients and/or adjuvants in the form of diluents, binders and lubricants such as, for example, amides, mannitol, cellulose etc., preferably precipitated silica and magnesium stearate.

Following stages 5 and 6, the granulate can be mixed, if desired, with a lubricant or other suitable antiadhesive such as, for example, talc, magnesium stearate, magnesium oxide, etc., preferably precipitated silica and titanium dioxide. The possible antiadhesives are used in a percentage of between 1% and 10% by weight, preferably between 2% and 5%. The dimensions of the lubricant particles correspond to those of the commercially available standard lubricants (0.001 to 75 µ).

The following examples are provided in order to illustrate better the present description without, however, limiting its scope.

### Example 1

### Protected microgranular SAMe

The preparation of this granulate is justified by the particular physical characteristics of the active component and its high instability to air (due to its deliquescence and heat instability). These characteristics make it difficult to handle and extremely sensitive to the ambient conditions in which it is processed.

The hygroscopicity of the starting material SAMe (s.m.) was determined as increase in weight, relative to the time zero, the relative content being given in p.a.:

| Days of exposure 75 %RH and 20 °C | T=0 2.00 g | % ionic SAMe T(0)= 52.1 | % adenine + adenosine T(0) = 0.4 | % MTA T(0)= 0.6 | % water (KF) T(0)= 1.5 |
|---|---|---|---|---|---|
| 1 | 2.40 g | 51.1 | 1.3 | 1.5 | 2.1 |
| 2 | 2.90 g | 50.3 | 2.4 | 1.9 | 2.9 |
| 3 | 3.50 g | 47.3 | 2.9 | 2.4 | 3.4 |
| 4 | 4.50 g | 45.1 | 3.2 | 3.0 | 3.8 |

As shown by the data given in the table, SAMe is a substance of high hygroscopicity and high instability.
Composition:
A) SAMe disulphate p-toluenesulphonate: 70.0 % by weight
B) Magnesium stearate: 0.5 % by weight
C) Precipitated silica: 2.0 % by weight
D) Hydrogenated vegetable fat: 27.5 % by weight Components A, B and part of component C are mixed in a SAGA rotating screw mixer for 15 minutes.

A Ronchi tablet compressing machine equipped with a forced-feeding hopper is charged with the mixture thus prepared. The machine is fitted with moulds for forming tablets having a diameter of 25 mm. This produces slugs having a hardness of 25/30 kp. At the end of the precompression stage, the tablets are introduced into a Viani vibrating granulator equipped with a perforated plate having 2 mm holes.

The granulate obtained is separated from its finest fractions by sieving through a Weston shaking sieve fitted with a 0.5 mm mesh.

The granulate thus selected is charged into a SAGA powder mixer equipped with a high-speed rotating screw and a cooling jacket. While continuing the vigorous stirring of the granulate charge, the melted vegetable fat is added at a temperature of 70°C using an airless gun and a suitable pumping system. The process temperature must be kept at 45°C ± 5°C. Any small agglomerates which may form can be separated off subsequently by sieving with meshes of suitable dimensions.

### Results of two production batches:

| Protected SAMe granulate batch TRA 001 | |
|---|---|
| Content: | 34.6 % |
| Humidity (KF): | 1.34 |
| Granule density: | 0.74 % |
| Particle size: | 500 - 2000 micron |
| Stability data: | |
| SAMe disulphate p-toluenesulphonate: | 33.10 % |
| Adenosine: | 2.20 % |
| Methylthioadenosine (MTA): | 2.40 % |

| Dissolution test in water (USP 711) | |
|---|---|
| Conditions: | 1.5 g of SAMe granules in 900 mL of distilled water 37±1°C at 150 rpm. 20 microlitres are filtered directly and injected directly into an HPLC. |

| Hours | 0.5 | 1.0 | 2.0 | 3.0 | 4.0 | 6.0 |
|---|---|---|---|---|---|---|
| | | | | | | |
| % SAMe | 10.5 | 27.9 | 52.7 | 68.9 | 78.4 | 85.3 |
| % SAMe | 10.1 | 26.7 | 50.8 | 66.6 | 75.4 | 84.7 |
| % SAMe | 9.4 | 28.5 | 51.2 | 66.9 | 76.4 | 85.9 |
| % SAMe | 9.7 | 25.9 | 52.3 | 69.4 | 75.9 | 86.5 |
| % SAMe | 9.6 | 27.6 | 53.2 | 70.3 | 79.3 | 88.5 |
| % SAMe | 10.3 | 25.8 | 49.9 | 67.8 | 80.5 | 91.3 |

| STABILITY OF THE PROTECTED PRODUCT TRA001, RELATIVE TO THE STARTING MATERIAL (s.m.) | | | | |
|---|---|---|---|---|
| | T=0 | T=0 | 5 days at 53 °C | 5 days at 53 °C |
| | s.m. | TRA00 1 | s.m. | TRA001 |
| % Ionic SAMe content | 52.1 | 33.10 | 48.5 | 32.17 |
| % adenine +adenosine | 0.4 | 0.5 | 3.35 | 2.20 |
| % MTA | 0.6 | 0.6 | 3.45 | 2.40 |
| % water(KF) | 1.5 | 1.3 | 1.5 | 1.3 |
| | | | | |
| Increase of hygroscopicity in % by weight | T=0 | T=0 | RH:75% t:40°C 1 day | RH:75% t:40 °C 1 day |
| 75 % RH 40 °C | 0.00 | 0.00 | 50 % | 5 % |

| Protected SAMe granules batch TRA 002 | |
|---|---|
| Content: | 35.5 % |
| Humidity (KF): | 1.44 |
| Granulate density: | 0.76 % |
| Particle size: | 500 - 2000 micron |
| Stability data: | |
| SAMe disulphate p-toluenesulphonate: | 33.60 % |
| Adenosine: | 2.36 % |
| Methylthioadenosine: | 2.72 % |

| Dissolution test in water (USP 711) | |
|---|---|
| Conditions: | 1.5 g of SAMe granules in 900 mL of distilled water 37±1°C at 150 rpm. 20 microlitres are taken and filtered and injected directly into an HPLC. |

| Hours | 0.5 | 1.0 | 2.0 | 3.0 | 4.0 | 6.0 |
|---|---|---|---|---|---|---|
| | | | | | | |
| % SAMe | 11.2 | 25.9 | 50.7 | 65.4 | 77.3 | 83.3 |
| % SAMe | 10.3 | 26.2 | 50.2 | 63.2 | 74.5 | 84.4 |
| % SAMe | 10.4 | 24.5 | 51.8 | 65.9 | 72.4 | 83.1 |
| % SAMe | 9.3 | 23.9 | 49.3 | 66.4 | 75.9 | 82.1 |
| % SAMe | 10.6 | 26.6 | 48.2 | 64.2 | 77.3 | 85.8 |
| % SAMe | 10.9 | 25.2 | 49.9 | 67.3 | 79.3 | 81.3 |

| STABILITY OF THE PROTECTED PRODUCT TRA002, RELATIVE TO THE STARTING MATERIAL (s.m.) | | | | |
|---|---|---|---|---|
| | T=0 | T=0 | 5 days at 53 °C | 5 days at 53 °C |
| | s.m | TRA002 | s.m. | TRA002 |
| % ionic SAMe content | 52.4 | 33.60 | 47.3 | 32.54 |
| % adenine + adenosine | 0.6 | 0.4 | 3.54 | 2.36 |
| % MTA | 0.3 | 0.5 | 3.87 | 2.72 |
| % water (KF) | 1.6 | 1.3 | 1.5 | 1.2 |
| | | | | |
| Increase of hygroscopicity in % by weight | T=0 | T=0 | 1 day at 75 % RH and 40 °C | 1 day at 75 % RH and 40 °C |
| 75 % RH 40 °C | 0.00 | 0.00 | 50 % | 5 % |

### Example 2

### Magnesium chloride hexahydrate

### Formulation:

Magnesium chloride hexahydrate: 70 % by weight
Precipitated silica: 2 % by weight
Hydrogenated vegetable fat:28 % by weight
For the production process, see Example 1

### Example 3

Proteolytic enzymes to be added to the detergents Formulation:
Proteolytic enzymes: 20 % by weight
Mannitol: 60 % by weight
Hydrogenated vegetable fat: 20% by weight

For the production process, see Example 1

### Example 4

### Lactic ferments

### Formulation:

Lactic ferments: 20% by weight
Mannitol: 60 % by weight
Hydrogenated vegetable fat:20 % by weight

### Example 5

### Amino acids in general and creatine in particular

### Formulation:

| | |
|---|---|
| Creatin | 40 % by weight |
| Lactose | 30 % by weight |
| Magnesium stearate | 1 % by weight |
| Colloidal silica | 1 % by weight |
| Hydrogenated vegetable fat | 28 % by weight |

For the production process, see Example 1

### Example 6

### Mixtures of branched amino acids

### Formulation:

| | |
|---|---|
| L-leucine | 25 % by weight |
| L-valine | 10 % by weight |
| L-isoleucine | 10 % by weight |
| Microcrystalline cellulose | 15 % by weight |
| Maltodextrin | 10 % by weight |
| Magnesium stearate | 2.5 % by weight |
| Talc | 2.5 % by weight |
| Hydrogenated vegetable fat | 25 % by weight |

For the production process, see Example 1

### Example 7

### Arginine

### Formulation:

| | |
|---|---|
| L-arginine | 50 % by weight |
| Mannitol | 10 % by weight |
| Microcrystalline cellulose | 10 % by weight |
| Lactose | 10 % by weight |
| Magnesium stearate | 1 % by weight |
| Colloidal silica | 1 % by weight |
| Hydrogenated vegetable fat | 18 % by weight |

For the production process, see Example 1

### Example 8

### Antioxidants in general

NADH, like all the antioxidants and active components in the previous examples, is also a highly hygroscopic and photosensitive substance.

The hygroscopicity and photosensitivity of the starting material NADH (s.m.) was determined as increase in weight, relative to time zero, the relative content being given in p.a.:

For the production process, see Example 1

| Days of exposure to light RH: 75% t: 20°C | T=0 2.00 g | T=0 NADH content 98.4% | T=0 NAD+ impurity 0.55% | T=0 Water (KF) 1.2% |
|---|---|---|---|---|
| 1 | 2.20 g | 97.9 | 1.21 | 1.82 |
| 2 | 2.40 g | 97.3 | 1.55 | 1.91 |
| 3 | 2.50 g | 96.0 | 2.89 | 2.44 |
| 4 | 2.80 g | 95.5 | 3.45 | 2.86 |

### Results:

### Protected NADH granulate:

### Formulation:

| | |
|---|---|
| NADH | 10 % by weight |
| Mannitol | 45 % by weight |
| Microcrystalline cellulose | 10 % by weight |
| Calcium carbonate | 10 % by weight |
| Colloidal silica | 1.5 % by weight |
| Magnesium stearate | 0.5 % by weight |
| Titanium dioxide | 3.0 % by weight |

Hydrogenated vegetable fat 20 % by weight

Results of two production batches:

Protected NADH granulate batch TRB 001

| | |
|---|---|
| Content: | 10.2 % |
| Humidity (KF): | 1.56 |
| Granulate density: | 0.81% |
| Particle size: | 500 - 2000 micron |

The product thus obtained is much less hygroscopic, less photosensitive and much more stable against atmospheric agents.

| STABILITY OF THE PROTECTED PRODUCT TRB001, RELATIVE TO THE STARTING MATERIAL | | | | |
|---|---|---|---|---|
| | T =0 | T = 0 | 5 days at 53 °C | 5 days at 53 °C |
| | Starting mat. | TRB001 | Starting mat. | TRB001 |
| % NADH content | 100.1 | 10.2 | 98.4 | 10.1 |
| % NAD+ content | 0.5 | 0.6 | 2.3 | 0.8 |
| % water (KF) | 1.3 | 1.1 | 1.5 | 1.2 |
| | | | | |
| Exposure to air of 5.0 grams | NADH content HPLC | NADH content HPLC | NADH content HPLC | NADH content HPLC |
| Days: | 1.0 | 2.0 | 4.0 | 7.0 |
| Protected granulate | 10.2 | 10.2 | 10.1 | 10.1 |
| Starting material | 100.0 | 98.0 | 91.0 | 89.1 |

### Dissolution test in water (USP 711)

Conditions: 1.5 g of NADH granules in 900 mL of a 0.1 M HCl solution pH 1.0 for one hour, 900 mL of sodium bicarbonate pH 8.5 containing 1.5 % of sodium lauryl sulphate for the remaining 7 hours, all temperature-regulated at 37±1°C at 100 rpm. 20 microlitres are taken and filtered and injected directly into an HPLC.

| Hours | 1.0 | 2.0 | 3.0 | 4.0 | 6.0 | 7.0 | 8.0 |
|---|---|---|---|---|---|---|---|
| % NADH | 8.9 | 36.3 | 44.8 | 56.4 | 63.2 | 69.3 | 77.9 |
| % NADH | 9.0 | 35.2 | 48.7 | 54.4 | 62.1 | 70.3 | 78.7 |
| % NADH | 8.6 | 37.4 | 46.8 | 53.8 | 63.2 | 71.2 | 80.3 |
| % NADH | 10.4 | 36.7 | 48.3 | 53.2 | 60.1 | 72.1 | 79.3 |
| % NADH | 9.6 | 35.9 | 45.7 | 54.7 | 65.3 | 70.3 | 81.2 |
| % NADH | 9.9 | 36.0 | 47.2 | 55.3 | 66.3 | 73.2 | 80.3 |

### Protected NADH granulate batch TRB 002 (same formulation)

| | |
|---|---|
| Content: | 10.5 % |
| Humidity (KF): | 1.73% |
| Granulate density: | 0.83 |
| Particle size range: | 500 - 2000 micron |

| 5 g exposed to air | Days 1.0 | Days 2.0 | Days 4.0 | Days 7.0 |
|---|---|---|---|---|
| | NADH content HPLC | NADH content HPLC | NADH content HPLC | NADH content HPLC |
| Protected granulate | 10.5 | 10.4 | 10.5 | 10.3 |
| Starting material | 10.2 | 9.9 | 9.2 | 8.3 |

### Dissolution test in water (USP 711)

Conditions: 1.5 g of NADH granulate in 900 mL of a 0.1 M HCl solution pH 1.0 for one hour, 900 mL of sodium bicarbonate pH 8.5 containing 1.5 % of sodium lauryl sulphate for the remaining 7 hours, all temperature-regulated at 37±1°C at 100 rpm. 20 microlitres are taken and filtered and injected directly into an HPLC.

| Hours | 1.0 | 2.0 | 3.0 | 4.0 | 6.0 | 7.0 | 8.0 |
|---|---|---|---|---|---|---|---|
| % NADH | 8.1 | 37.3 | 46.8 | 58.9 | 66.2 | 74.3 | 83.9 |
| % NADH | 9.1 | 38.7 | 49.7 | 57.7 | 68.6 | 75.7 | 84.7 |
| % NADH | 8.2 | 39.4 | 50.8 | 58.8 | 68.5 | 77.5 | 85.3 |
| % NADH | 10.4 | 38.7 | 50.2 | 56.7 | 67.1 | 76.1 | 84.4 |
| % NADH | 10.6 | 37.9 | 47.7 | 587 | 69.3 | 77.5 | 85.6 |
| % NADH | 10.9 | 38.0 | 49.5 | 59.7 | 67.3 | 75.2 | 87.4 |

### Example 9

### GSH (reduced glutathione)

### Formulation:

| | |
|---|---|
| GSH | 50 % by weight |
| Glyceryl behenate | 5.0 % by weight |
| Microcrystalline cellulose | 10 % by weight |
| Mannitol | 12.5 % by weight |
| Colloidal silica | 2.0 % by weight |
| Magnesium stearate | 0.5 % by weight |
| Hydrogenated vegetable fat | 20.0 % by weight |

For the production process, see Example 1

## Claims

1. Granular formulation based on granules formed by an inner core essentially consisting of at least a salt of SAMe chosen from among disulphate ditosylate, disulphate para-toluenesulphonate, 1,4-butanedisulphonate and/or hexamethanephosphonate in an amount of between 50% and 90% by weight of the formulation, and by an outer coating essentially consisting of hydrogenated vegetable fats in an amount of between 20% and 40% by weight of the formulation.

2. Granular formulation according to Claim 1, in which said active component is present in an amount of between 60% and 80% by weight.

3. Granular formulation according to claim 1, in which said salt of SAMe is non-palatable, hygroscopic, oxidisable and/or photosensitive.

4. Granular formulation according to claim 1, in which the hydrogenated vegetable fats are chosen from among: mono-, di-, triglyceryl behenates, glyceryl palmitostearate, polyethylene glycol esters of fatty acids and/or waxes.

5. Granular formulation according to claim 1, in which the hydrogenated vegetable fats have a melting point of between 30° and 100°C.

6. Granular formulation according to claim 5, in which the hydrogenated vegetable fats have a melting point of between 50° and 80°C.

7. Granular formulation according to claim 1, in which the hydrogenated vegetable fats are hydrogenated fatty acids or mixtures thereof having chains of 14 to 22 carbon atoms.

8. Granular formulation according to claim 1, in which the granules have a particle size of between 250 and 3000 µ.

9. Granular formulation according to claim 8, in which the granules have a particle size of between 250 and 1000 µ.

10. Granular formulation according to claim 1, **characterized in that** it contains pharmaceutically acceptable excipients and/or adjuvants in an amount of between 2% and 10%, relative to the weight of the formulation.

11. Formulation according to Claim 10, in which said excipients and/or adjuvants are present in an amount of between 3% and 5%, relative to the weight of the formulation.

12. Mixture for veterinary use containing feed and a formulation according to any of the preceding claims.

## Patentansprüche

1. Körnige Formulierung, basierend auf Körnchen, die gebildet sind aus einem inneren Kern, der im Wesentlichen aus mindestens einem Salz von SAMe besteht, ausgewählt aus Disulfatditosylat, Disulfatpara-toluolsulfonat, 1,4-Butandisulfonat und/oder Hexamethanphosphonat, in einer Menge zwischen 50 und 90 Gew.% der Formulierung, und einer äußeren Beschichtung, die im Wesentlichen aus hydrierten Pflanzenfetten besteht, in einer Menge zwischen 20 und 40 Gew.% der Formulierung.

2. Körnige Formulierung gemäß Anspruch 1, worin die aktive Komponente in einer Menge von 60 bis 80 Gew.% vorliegt.

3. Körnige Formulierung gemäß Anspruch 1, worin das Salz von SAMe nicht genießbar, hygroskop, oxidierbar und/oder lichtempfindlich ist.

4. Körnige Formulierung gemäß Anspruch 1, worin die hydrierten Pflanzenfette ausgewählt sind aus: Mono-, Di-, Triglycerylbehenaten, Glycerinpalmitostearat, Polyethylenglykolestern von Fettsäuren und/oder Wachsen.

5. Körnige Formulierung gemäß Anspruch 1, worin die hydrierten Pflanzenfette einen Schmelzpunkt zwischen 30 und 100°C aufweisen.

6. Körnige Formulierung gemäß Anspruch 5, worin die hydrierten Pflanzenfette einen Schmelzpunkt zwischen 50 und 80°C aufweisen.

7. Körnige Formulierung gemäß Anspruch 1, worin die hydrierten Pflanzenlette hydrierte Pflanzenfette oder Mischungen hiervon sind, die Ketten von 14 bis 22 Kohlenstoffatomen aufweisen.

8. Körnige Formulierung gemäß Anspruch 1, worin die Körnchen eine Partikelgröße zwischen 250 und 3000 µm aufweisen.

9. Körnige Formulierung gemäß Anspruch 8, worin die Körnchen eine Partikelgröße von 250 bis 1000 µm aufweisen.

10. Körnig Formulierung gemäß Anspruch 1, **dadurch gekennzeichnet, dass** sie pharmazeutisch akzeptable Exzipienten und/oder Adjuvanzien in einer Menge von 2 bis 10% enthält, relative zum Gewicht der Formulierung.

11. Formulierung gemäß Anspruch 10, worin die Exzipienten und/oder Adjuvanzien in einer Menge zwischen 3 und 5% vorliegen, relative zum Gewicht der Formulierung.

12. Mischung für die tiermedizinische Verwendung, enthaltend Futterstoffe und eine Formulierung gemäß einem der vorstehenden Ansprüche.

## Revendications

1. Formulation granulaire à base de granules constitués d'un noyau interne essentiellement constitué d'au moins un sel de SAMe choisi parmi le ditosylate de disulfate, le para-toluènesulfonate de disulfate, le 1,4-butanedisulfonate et/ou l'hexaméthanephosphonate en une quantité comprise entre 50 % et 90 % en poids de la formulation, et d'une couche externe essentiellement constituée de graisses végétales hydrogénées en une quantité comprise entre 20 % et 40 % en poids de la formulation.

2. Formulation granulaire selon la revendication 1, dans laquelle ledit composant actif est présent en une quantité comprise entre 60 % et 80 % en poids.

3. Formulation granulaire selon la revendication 1, dans laquelle ledit sel de SAMe est inappétent, hygroscopique, oxydable et/ou photosensible.

4. Formulation granulaire selon la revendication 1, dans laquelle les graisses végétales hydrogénées sont choisies parmi : les béhénates de mono-, di- et triglycéryle, le palmitostéarate de glycéryle, les esters de polyéthylène glycol d'acides gras et/ou les cires.

5. Formulation granulaire selon la revendication 1, dans laquelle les graisses végétales hydrogénées ont un point de fusion compris entre 30 et 100 °C.

6. Formulation granulaire selon la revendication 5, dans laquelle les graisses végétales hydrogénées ont un point de fusion compris entre 50 et 80 °C.

7. Formulation granulaire selon la revendication 1, dans laquelle les graisses végétales hydrogénées sont des acides gras hydrogénés ou des mélanges de ceux-ci comportant des chaînes de 14 à 22 atomes de carbone.

8. Formulation granulaire selon la revendication 1, dans laquelle les granules ont une granulométrie comprise entre 250 et 3 000 µm.

9. Formulation granulaire selon la revendication 8, dans laquelle les granules ont une granulométrie comprise entre 250 et 1 000 µm.

10. Formulation granulaire selon la revendication 1, **caractérisée en ce qu'**elle contient des excipients et/ou des adjuvants pharmaceutiquement acceptables en une quantité comprise entre 2 % et 10 % par rapport au poids de la formulation.

11. Formulation selon la revendication 10, dans laquelle lesdits excipients et/ou adjuvants sont présents en une quantité comprise entre 3 % et 5 % par rapport au poids de la formulation.

12. Mélange à usage vétérinaire contenant un aliment et une formulation selon l'une quelconque des revendications précédentes.
